(19)

**Europäisches Patentamt**
**European Patent Office**
**Office européen des brevets**

(11) **EP 2 275 028 A1**

(12) **EUROPEAN PATENT APPLICATION**

(43) Date of publication:
**19.01.2011 Bulletin 2011/03**

(51) Int Cl.:
*A61B 5/053* *(2006.01)*

(21) Application number: **09165509.2**

(22) Date of filing: **15.07.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(71) Applicants:
• **Koninklijke Philips Electronics N.V.**
**5621 BA Eindhoven (NL)**

• **Philips Intellectual Property & Standards**
**20099 Hamburg (DE)**

(72) Inventor: **The designation of the inventor has not yet been filed**

(74) Representative: **Damen, Daniel Martijn**
**Philips**
**Intellectual Property & Standards**
**P.O. Box 220**
**5600 AE Eindhoven (NL)**

(54) **Device, system, method and computer program for enabling a bioimpedance measurement**

(57) The invention relates to a bio impedance measuring device for measuring the bioimpedance of a portion of a body, the device comprising:
- at least one transmitter (24) for applying a perturbing signal at different frequencies (fl, f2) to the portion of the body; and
- at least one sensor (12, 27) for detecting a response signal (V1, V2) from the body related to said bioimpedance.

According to the invention the measuring device is a multiple frequency differential measuring device (10) for determining the bioimpedance from at least one difference (ΔV) between at least two detected response signals with a first response signal (V1) at a first frequency (fl) and a second response signal (V2) at a second frequency (f2).

The invention further relates to a monitoring system, a method of enabling a bioimpedance measurement of a portion of a body and a corresponding computer program.

Fig. 2

**Description**

FIELD OF THE INVENTION

The present invention relates to a bioimpedance measuring device for measuring the bioimpedance of a portion of a body, the device comprising at least one transmitter for applying a perturbing signal at different frequencies to the portion of the body, the perturbing signal comprising an alternating magnetic field; and at least one sensor for detecting response signals from the body related to said bioimpedance.

The present invention further relates to a monitoring system.

The present invention still further relates to a method of enabling a bioimpedance measurement of a portion of a body, said method comprising the steps of providing a device comprising at least one transmitter for applying a perturbing signal at different frequencies to the portion of the body and at least one sensor for detecting response signals from the body related to the said bioimpedance.

The present invention further relates to a corresponding computer program.

BACKGROUND OF THE INVENTION

The measurement of the bioimpedance is a known method to measure in a non-contact way various vital parameters of a human body.

The operating principle is the following: using an inductor loop of the transmitter, an alternating magnetic field is induced in a tissue of the human body. This alternating magnetic field causes eddy currents in the tissue of the body. Depending on the type of tissue, these eddy currents are stronger or weaker and cause losses in the tissue, which can be measured as a decrease of the quality factor of the inductor loop. They also cause a secondary magnetic field, which can be measured as an inductivity change of the inductor loop or as an induced voltage V1 of an appropriate frequency f1 in a second inductor loop (or pickup coil). The principle is similar to metal detection devices.

The measurement of the bioimpedance has been shown to allow the non-contact detection of several parameters like breath action and depth, heart rate and change of the heart volume. In the measurements of these parameters moving artifacts play a large role.

SUMMARY OF THE INVENTION

It is an object of the invention to provide a simple, yet safe and reliable bioimpedance measurement device as well as a corresponding system, method and computer program, which are not susceptible to moving artifacts.

To achieve this object, the measuring device is a multiple frequency differential measuring device for determining the bioimpedance from at least one difference $\Delta V$ between at least two detected response signals with a first response signal V1 at a first frequency f1 and a second response signal V2 at a second frequency f2. Since the response signals (e.g. three response signals at 1 MHz, 5 MHz and 10 MHz respectively) qualitatively show the same artifact, the differential signal(s) being the difference $\Delta V$ (or $\Delta V_1$, ..., $\Delta V_{n-1}$ respectively) between the measured response signals V1, ..., Vn shows a reduced artifact structure. The artifacts typically are artifacts caused by a movement of the body relatively to the bioimpedance measuring device. The bioimpedance especially is determined from weighted differences, wherein the differences $\Delta V_1$, ..., $\Delta V_{n-1}$ are weighted by predetermined weighting factors.

Especially, the (spectroscopic) measurements are done with a simple resonant system consisting of a single coil with at least one tuneable resonant capacitor. In addition, this tuneable resonant system does not only measure at distinct frequencies but allows easily and cost effective continuous frequency scans, which will improve the compensating effect.

The general technical measure of the invention is based on the insight that contactless non-invasive methods for measurements of a body signal allow an easy and comfortable means of controlling vital parameters, like heart rate, tissue water content and blood glucose level, notably to supervise the user without the need of applying any kind of invasive devices to the user's body. According to the technical measure of the invention a sole sensor operating in a resonant circuit provides a simple, reliable and low cost solution for spectroscopic measurement system of the bioimpedance. For distinguishing between different tissue classes, spectroscopic measurements are performed by changing the resonant frequency of the resonant circuit and by applying the alternating magnetic field to the portion of the body with the said changed frequency.

Especially the measuring device is a dual frequency differential measuring device for determining the bioimpedance from the difference $\Delta V$ between two detected response signals with the first response signal V1 at the first frequency f1 and the second response signal V2 at the second frequency f2.

Measurement of the bioimpedance is known per se. The operating principle is as follows: using an inductor loop an alternating magnetic field is induced in a portion of a body, notably a patient. This alternating magnetic field causes eddy currents in the tissue of the body. Depending on the type of tissue, these eddy currents are stronger or weaker. These eddy currents cause losses in the tissue, which can be measured as a decrease of the quality factor of the inductor loop. The quality factor is a measure of the time of free oscillations of the resonant circuit. The eddy currents also cause a secondary magnetic loop or an induced voltage in a second inductor loop.

Preferably, for changing the resonant frequency of the resonant circuit, a value of the capacitance integrated in the resonant circuit is changed. This can be done, for example, by arranging a plurality of different capacitors and by selecting a value of the net capacitance by operating a suitable switch. Alternatively, it is possible to add some length of connecting tracks, such that their parasitic capacity adds to the resonant capacity. Preferably, a tunable capacitor is used or a ceramic capacitor having non-linear dielectric properties. The tunable capacitor is preferably realized using a semiconductor diode and varying its DC offset.

The monitoring system according to the invention comprises the device as is described with reference to the foregoing.

According to a preferred embodiment of the invention, the at least one sensor is a sensor for detecting the at least two response signals V1,..., Vn simultaneously or sequentially in real-time or sequentially in near real-time. The decisive factor is the ratio between the timescale of the measurement of the period between the measurements of the two response signals and the timescale of the signal changes due to the artifact. The timescale of the measurement is for example given by a duty cycle of a corresponding computer.

According to another preferred embodiment of the invention, the bioimpedance measuring device comprises at least two sensors, which sensors are arranged at a distance equal to or less than 25% of the average dimension of the sensors to each other, wherein the first sensor detects the first response signal at the first frequency and the second sensor detects the second response signal at the second frequency. Preferably, the distance is equal to or less than 5% of the average dimension.

According to a preferred embodiment of the invention, the bioimpedance measuring device further comprises a determining unit for determining the bioimpedance from the difference $\Delta V$ between the two measured response signals V1, V2.

According to another preferred embodiment of the invention, the bioimpedance measuring device further comprises a controller means for controlling the transmitter and/or the sensor.

Preferably, a perturbing signal S comprises an alternating magnetic field.

According to yet another preferred embodiment of the invention, the sensor comprises a coil integrated in a resonant circuit with adjustable resonant frequency.

Preferably, the resonant circuit comprises a variable capacitance.

According to another preferred embodiment of the invention, the resonant circuit comprises a ceramic capacitor with non linear dielectric properties.

The invention further relates to a monitoring system for monitoring a vital sign of an individual, the system comprising an aforementioned device. Preferably, the sensor is integrated in a wearable article.

The invention further relates to a method of enabling a bioimpedance measurement of a portion of a body, said method comprising the steps of:

- providing a device comprising at least transmitter for applying a perturbing signal at different frequencies to the portion of the body and at least sensor for detecting a response signal from the body related to the said bioimpedance. According to the invention, the perturbing signal comprising an alternating magnetic field, the sensor being arranged as a coil integrated in a resonant circuit with adjustable resonant frequency;
- positioning the device in a vicinity of the portion of the body;
- applying a first alternating electromagnetic field at a first frequency f1 to said portion and detecting respective first response signal;
- applying a second alternating electromagnetic field at a second frequency f2 to said portion and detecting a respective second response signal; and
- determining the bioimpedance from the difference between the detected response signals. Preferably, the at least one sensor detects the two response signals V1, V2 simultaneously or sequentially in real-time or sequentially in near real-time.

Preferably the bioimpedance is determined from at least one difference $\Delta V$ between at least two detected response signals with a first response signal V1 at a first frequency f1 and a second response signal V2 at a second frequency f2.

The invention further relates to a computer program for use in the aforementioned method, wherein said computer program comprises computer instructions to determine the bioimpedance from the difference between the detected response signals.

The technical effects necessary according to the invention can thus be realized on the basis of the instructions of the computer program in accordance with the invention. Such a computer program can be stored on a carrier such as a CD-ROM or it can be available over the internet or another computer network. Prior to execution, the computer program is loaded into the computer by reading the computer program from the carrier, for example by means of a CD-ROM player, or from the internet, and storing it in the memory of the computer. The computer includes inter alia a central processor unit (CPU), a bus system, memory means, e. g. RAM or ROM, storage means, e. g. floppy disk or hard disk units and input/output units. In one embodiment the computer is part of a corresponding bioimpedance measuring device, especially part of a determining unit; in another embodiment the computer is adapted to cooperate with the bioimpedance measuring device, especially with a determining unit of the a bioimpedance measuring device, wherein the bioimpedance measuring device and the computer are parts of a measuring system.

According to a preferred embodiment of the invention said computer program comprises computer instructions for controlling at least one transmitter for applying the signals at the different frequencies and/or at least one detector for detecting the response signals.

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

BRIEF DESCRIPTION OF THE DRAWINGS

Additional details, features, characteristics and advantages of the object of the invention are disclosed in the subclaims, the figures and the following description of the respective figure and examples, which -in an exemplary fashion- show embodiments and examples of measuring devices according to the invention.

In the drawings:

Fig. 1 presents a schematic view of a first embodiment of a resonant bioimpedance measuring device according to the invention;

Fig. 2 presents a schematic view of a second embodiment of a resonant bioimpedance measuring device according to the invention;

Fig. 3 presents a schematic view of a third embodiment of a resonant bioimpedance measuring device according to the invention;

Fig. 4 presents a diagram showing a first detected response signal, a second detected response signal and the difference between the two response signals vs. time; and

Fig. 5 presents a diagram showing a comparison between the first measured response signal and a corresponding pressure curve.

DETAILED DESCRIPTION OF EMBODIMENTS

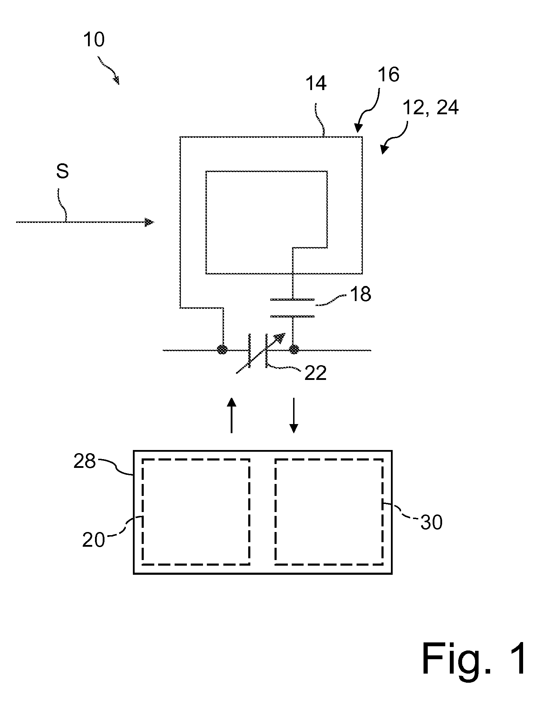

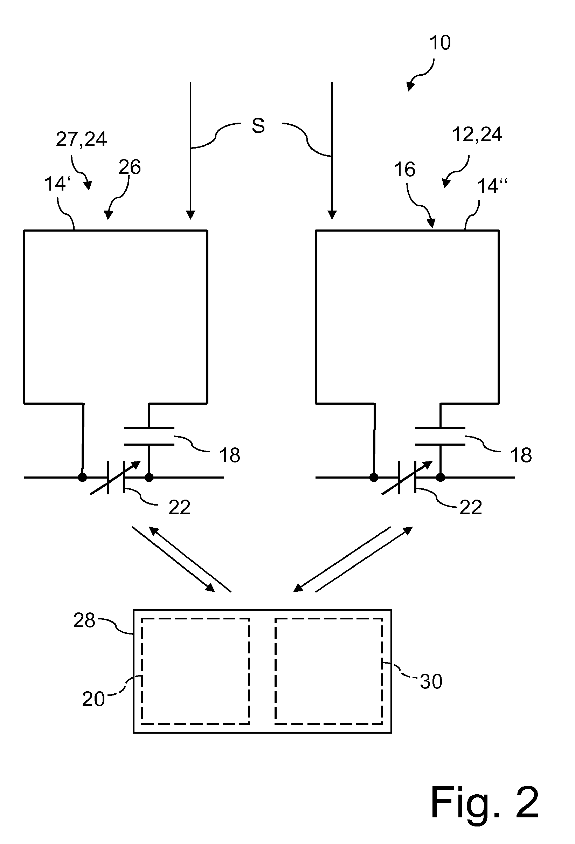

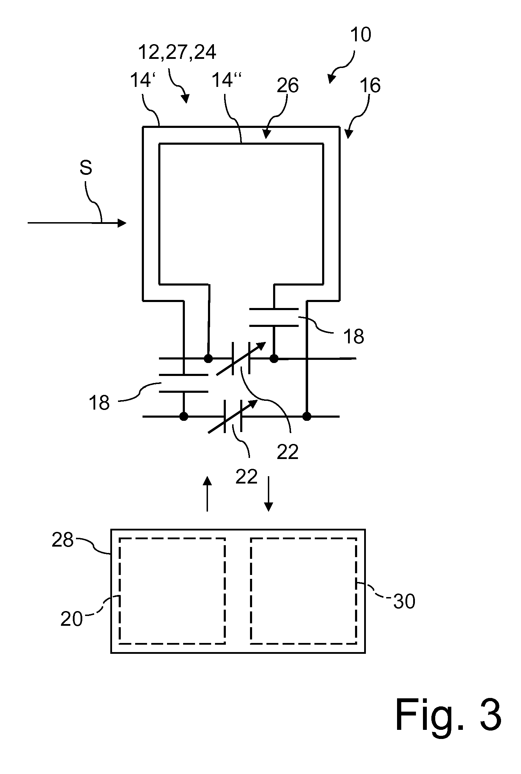

Fig. 1 shows a schematic view of a resonant bioimpedance measuring device 10 according to a first embodiment of the invention comprising one sensor 12. Each of the bioimpedance measuring device 10 shown in Figs. 2 and 3 comprises two sensors 12, 27 having almost the same position.

The resonant bioimpedance measuring device 10 shown in Fig. 1 comprises a sensor 12 having a coil 14, which coil 14 is preferably implemented as a planar spiral coil. More preferably the planar spiral coil 14 is integrated on a printed circuit board (not shown) or on any other suitably selected isolating material. The sensor 12 is realized as a resonant circuit 16 by means of electrically connecting the coil 14 to a DC blocking capacitance 18. In order to realize a measurement using different resonating frequencies f1 and f2 the bioimpedance measuring device 10 is provided with a controller means 20, which causes a change in the resonant frequency of the resonant circuit 16 by changing a value of a variable total capacitance. This is done, for example, by arranging a plurality of different capacitors and by selecting a value of a net capacitance by operating a suitable switch. Alternatively, it is possible to add some length of connecting tracks, such that their parasitic capacity adds to the entire capacity of the resonant circuit. Preferably, a variable capacitor 22 is used or a ceramic capacitor having non-linear dielectric properties. The controller means 20 is tuning the variable capacitor 22 for changing the resonating frequency between at least two different frequencies f1 and f2 which are respective resonance frequencies due to the value of the total capacitance. Another preferred solution is realized by using a semiconductor diode and varying its DC offset.

The controller means 20 further controls a transmitter of the bioimpedance measuring device 10. In this embodiment the coil 14 is part of a transmitter 24 and the sensor 12. The transmitter 24 uses the coil 14 as an inductor loop for inducing an alternating magnetic field in a tissue of the human body. The bioimpedance measuring device 10 is arranged in such a way that the coil 14 (as the coil of the sensor 12) is conceived to detect a response signal (arrow S) of the portion of the body.

Alternatively, the bioimpedance measuring device 10 may be provided with a further resonant circuit 26 to measure the induced voltage in response to alternating magnetic field applied by the resonating circuit 16. The controller means 20 further controls said transmitter 24 of the bioimpedance measuring device 10.

Fig. 2 shows the measuring device 10 with the resonance circuit 16 of the sensor 12 arranged on one side of a plate- or film-like substrate (for example the board or card of a printed circuit board) and a further resonance circuit 26 of a further sensor 27 arranged on the opposite side wherein at least the coils 14', 14" of the sensors 12, 27 are arranged congruent or at least substantially congruent in a top view.

The resonance circuit 16 of the sensor 12 has an adjustable first resonance frequency f1 and the further resonance circuit 26 of the further sensor 27 has a second resonance frequency f2. Preferably the first resonance frequency f1 of the sensor 12 and the second resonance frequency f2 of the further sensor 27 differ to such an extent, that they do not interact or at least are not equal. If they interact and change from f1 to f1' and f2 to f2' due to the interaction, it is necessary that f1' ≠ f2'.

Fig. 3 shows the measuring device 10 with the resonance circuits 16, 26 of the sensor 12 and the further sensor 27 arranged on one side of the plate- or film-like substrate, wherein at least one of the coils 14', 14" (the coil 14' of the sensor 27 or the coil 14" of the further sensor 12) is arranged inside the other coil 14", 14' (the coil 14" of the sensor 12 or the coil 14' of the further sensor 27).

Preferably, the dimensions of the coils 14', 14" deviate less than 20% of each other, more preferably the dimensions of the coils 14', 14" deviate less than 10% of each other, especially the dimensions of the coils 14', 14" deviate less than 5% of each other.

Especially, the distance of the conductors of the coils 14', 14" preferably is equal to or less than five times of the average conductor width, more preferably equal to or less than two times of the average conductor width of said conductors constituting the coil 14', 14".

The controller means 20 is part of a determining device 28. The determination device 28 further comprises a determining unit 30 for determining the bioimpedance from the difference ΔV between (at least) two measured response signals V1, V2 with a first response signal V1 measured with a first frequency f1 and a second response signal V2 measured with a second frequency f2. Therefore the resonant bioimpedance measuring device 12 according to a preferred embodiment is a dual frequency differential measuring device.

According to the technical measure of said embodiment of the invention a sole coil operating in a resonant circuit with variable resonant frequencies provides a simple, reliable and low cost solution for measurement system of the bioimpedance.

For distinguishing between different tissue classes spectroscopic measurements are performed by changing the resonant frequency of the resonant circuit and by applying the alternating magnetic field to the portion of the body with the said changed frequency.

Preferably, the bioimpedance measuring device 10 is integrated in a monitoring system for monitoring a vital sign of an individual.

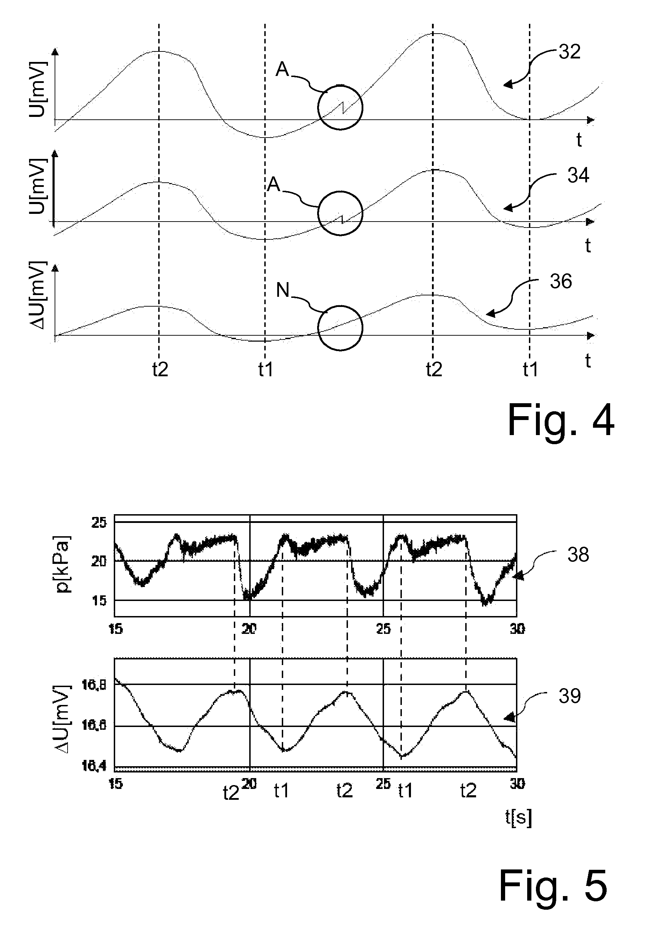

In Fig. 4 an example of a first measured response signal V1 at a first frequency f1 corresponding to a breathing signal is shown as a first graph 32 in the top view, a second measured response signal V2 at a second frequency f2 corresponding to the same breathing signal is shown as a second graph 34 in the mid view and the difference ΔV between the two measured response signals (first response signal V1 at a first frequency f1 and second response signal V2 at a second frequency f2) is shown as a third graph 36 in the bottom view. Each of the first and second graph 32, 34 of the measured response signals V1, V2 (top view, mid view) shows an artifact A, which is situated between a first measuring point t1 and a second measuring point t2. Within the third graph 36, which represents the difference ΔV between the two measured response signals, the artifacts are averaged out (section N of the graph is devoid of artifact structures).

Table 1 gives the conductivity values σ and relative permittivity values $\varepsilon_r$ for the different tissue classes: blood, lung (deflated), lung (inflated) and fat.

Table 1: conductivity σ and relative permittivity $\varepsilon_r$ for different tissue classes

| Frequ. [MHz] | Blood | | Lung_deflated | | Lung_inflated | | Fat | |
|---|---|---|---|---|---|---|---|---|
| | conduct. σ [S/m] | relative permit. $\varepsilon_r$ | conduct. σ [S/m] | relative permit. $\varepsilon_r$ | conduct. [S/m] | relative permit. $\varepsilon_r$ | conduct. σ [S/m] | relative permit. $\varepsilon_r$ |
| 1.0 | 0.82211 | 3026.3 | 0.33438 | 1170.5 | 0.13609 | 733.18 | 0.025079 | 27.222 |
| 2.0 | 0.92605 | 1680.7 | 0.36685 | 659.41 | 0.15826 | 491.16 | 0.025501 | 22.952 |
| 3.0 | 0.98268 | 1080 | 0.3855 | 462.21 | 0.17526 | 363.54 | 0.02595 | 20.917 |
| 4.0 | 1.0169 | 773.45 | 0.39826 | 361.27 | 0.18799 | 284.96 | 0.026427 | 19.429 |
| 5.0 | 1.04 | 596.12 | 0.40796 | 300.68 | 0.19771 | 233.12 | 0.026918 | 18.181 |
| 6.0 | 1.0568 | 483.57 | 0.41583 | 260.42 | 0.20538 | 197.05 | 0.027405 | 17.087 |
| 7.0 | 1.0699 | 407.07 | 0.42247 | 231.77 | 0.21163 | 170.85 | 0.027878 | 16.113 |
| 8.0 | 1.0804 | 352.29 | 0.42825 | 210.32 | 0.21686 | 151.12 | 0.028329 | 15.242 |
| 9.0 | 1.0892 | 311.46 | 0.43337 | 193.66 | 0.22134 | 135.82 | 0.028754 | 14.464 |
| 10.0 | 1.0967 | 280.03 | 0.43799 | 180.32 | 0.22524 | 123.66 | 0.029152 | 13.767 |

Fig. 5 shows a comparison between the first measured response signal V1, which is the induced voltage at the sensor 12 at a first frequency f1, and the corresponding pressure curve 38. The conductivity of the thorax decreases during inspiration (time interval between t2 and t1, wherein the lung is deflated) and increases during expiration (time interval between t1 and t2, wherein the lung is inflated).

The difference $\Delta'V1$ of the first measured response signal data V1 between the measuring points t1 and t2 is:

$$\Delta'V1 = V1(t1) - V1(t2) = G_{body,\,t1} * \sigma_{f1,\,fat} + G_{lung,\,t1} * \sigma_{f1,\,lung\_inflated} - [G_{body,\,t2} * \sigma_{f1,\,fat} + G_{lung,\,t2} * \sigma_{f1,\,lung\_deflated}] \qquad (1)$$

Where $G_{body}$, $G_{lung}$ are geometrical factors and $\sigma$ are the conductivity values, which are depended of the tissue and the frequency. If now a second measurement is carried out at a second frequency f2 the conductivity values $\sigma$ change.

In case of a moving artefact $G_{body}$ and $G_{lung}$ are changing, which is shown by the incise t1 and t2.

The difference $\Delta'V2$ of the second measured response signal data V1 between the measuring points t1 and t2 is:

$$\Delta'V2 = V2(t1) - V2(t2) = G_{body,\,t1} * \sigma_{f2,\,fat} + G_{lung,\,t1} * \sigma_{f2,\,lung\_inflated} - [G_{body,\,t2} * \sigma_{f2,\,fat} + G_{lung,\,t2} * \sigma_{f2,\,lung\_deflated}] \qquad (2)$$

Since the second signal at f2 shows the same artefact, the influence of the geometrical factors will almost disappear, wherein with $\Delta'(\Delta V) = \Delta'V1 - \Delta'V2$

$$\Delta'(\Delta V) = (\Delta' G_{body}) * (\Delta\sigma_{fat}) + G_{lung,\,t1} * (\Delta\sigma_{lung\_inflated}) - G_{lung,\,t2} * (\Delta\sigma_{lung\_deflated}) \qquad (3)$$

applies.

By subtraction of the measured response signals the geometrical factors and therefore their influence can mostly be eliminated, which can be represented e.g. in the bottom view (3$^{rd}$ graph 36) of Fig. 4. With the artifact determined signal being smaller than the real response signal:

$$\Delta' G_{lung} \ll (\Delta\sigma_{lung\_inflated} - \Delta\sigma_{lung\_deflated})$$

the change of the geometrical factor $G_{lung}$ is negligible ($G_{lung,\,t1} \approx G_{lung,\,t2}$) with respect to the change of the corresponding conductivity $\sigma_{lung}$ and

$$\Delta'(\Delta V) \approx (\Delta' G_{body}) * (\Delta\sigma_{fat}) + G_{lung,\,t2} * (\Delta\sigma_{lung\_t1} - \Delta\sigma_{lung\_t2}) \qquad (4)$$

applies.

The resulting first term of eq. (4) is a simple offset and the second term is almost the real response signal due to the change of $\sigma_{lung}$.

In this described procedure measurements at two distinct frequencies f1, f2 are used for the compensation. The invention allows an easy and fast measurement of a whole spectrum, which means a number of frequency points. By using a least square algorithm all frequency point scan be considered for the artefact compensation, which improves the compensation quality over the existing method.

Measurements of bioimpedance has been shown to allow a non-contact detection of several vital parameters, like breath action and depth, heart rate, change of the heart volume and the blood glucose level. Further parameters that can be measured using the bioimpedance measurement are fat or water content of the tissue. The monitoring system according to an embodiment of the invention is preferably arranged to measure a signal representative of these vital parameters. Usually, it is not sufficient to measure bioimpedance with one frequency and it is important to use setup to measure with different frequencies. Such setup has an additional advantage, because if the difference between two frequencies is measured, the measurement is not as dependent on exact positioning as it is for a single frequency measurement. Therefore, the monitoring system according to the invention is simple, low cost and reliable in use and can easily be used to monitor any relevant vital sign, like ones mentioned above, of the individual. Preferably, for durable measurements, the monitoring system according to a preferred embodiment is integrated into a wearable article, like an elastic belt, wrist watch, or piece of clothing in tight contact with the body.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

**Claims**

1.  A bioimpedance measuring device (10) for measuring the bioimpedance of a portion of a body, the device comprising:

    - at least one transmitter (24) for applying a perturbing signal at different frequencies (f1, f2) to the portion of the body, the perturbing signal comprising an alternating magnetic field; and
    - at least one sensor (12, 27) for detecting response signals (V1, V2) from the body related to said bioimpedance;

    wherein the measuring device (10) is a multiple frequency differential measuring device (10) for determining the bioimpedance from at least one difference ($\Delta V$) between at least two detected response signals with a first response signal (V1) at a first frequency (f1) and a second response signal (V2) at a second frequency (f2).

2.  The device according to claim 1, wherein the at least one sensor (12, 27) is a sensor for detecting the response signals (V1, V2) simultaneously or sequentially in real-time or sequentially in near real-time.

3.  The device according to claim 1, wherein the bioimpedance measuring device (10) comprises at least two sensors, which sensors (12, 27) are arranged at a distance equal to or less than 25% of the average dimension of the sensors (12, 27) to each other, wherein the first sensor (12) detects the first response signal at the first frequency (f1) and the second sensor (27) detects the second response signal at the second frequency (f2).

4.  The device according to claim 1, wherein the bioimpedance measuring device (10) further comprises a determining unit (30) for determining the bioimpedance from the difference ($\Delta V$) between the measured response signals (V1, V2).

5.  The device according to claim 1, wherein the bioimpedance measuring device (10) further comprises a controller means (18) for controlling the transmitter (24) and/or the sensor (12, 27).

6.  The device according to claim 1, wherein the sensor (12, 27) and/or the transmitter (24) comprises a coil (14, 14', 14") integrated in a resonant circuit (16, 26) with adjustable resonant frequency.

7.  The device according to claim 6, wherein the resonant circuit (16, 26) comprises a variable capacitance (20).

8.  The device according to claim 6, wherein the resonant circuit (16, 26) comprises a ceramic capacitor with non linear dielectric properties.

9.  A monitoring system for monitoring a vital sign of an individual, the system comprising a bioimpedance measuring device (10) according to claim 1.

10. A monitoring system according to claim 9, wherein the sensor (12, 27) and/or the transmitter (24) is integrated in a wearable article.

11. A method of enabling a bioimpedance measurement of a portion of a body, said method comprising the steps of:

    - providing a device comprising at least one transmitter for applying a perturbing signal at different frequencies to the portion of the body and at least one sensor for detecting response signals from the body related to the said bioimpedance, the perturbing signal comprising an alternating magnetic field, the sensor being arranged as a coil integrated in a resonant circuit with adjustable resonant frequency;

- positioning the device in a vicinity of the portion of the body;

- applying a first alternating electromagnetic field at a first frequency to said portion and detecting respective first response signal;

- applying a second alternating electromagnetic field at a second frequency to said portion and detecting a respective second response signal; and

- determining the bioimpedance from the difference between the detected response signals.

**12.** The method according to claim 11, wherein the at least one sensor detects the response signals simultaneously or sequentially in real-time or sequentially in near real-time.

**13.** A computer program for use in the method according to claim 11, wherein said computer program comprises computer instructions to determine the bioimpedance from the difference ($\Delta V$) between the detected response signals (V1, V2).

**14.** The computer program according to claim 13, wherein said computer program comprises computer instructions for controlling:

- at least one transmitter (24) for applying the signals at the different frequencies (f1, f2) and/or
- at least one detector (12, 27) for detecting the response signals (V1, V2).

Fig. 1

Fig. 2

Fig. 3

Fig. 4

Fig. 5

# EP 2 275 028 A1

| | Europäisches Patentamt European Patent Office Office européen des brevets | **EUROPEAN SEARCH REPORT** | | Application Number EP 09 16 5509 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2008/029316 A (PHILIPS INTELLECTUAL PROPERTY [DE]; KONINK PHLIPS ELECTRONICS N V [NL]) 13 March 2008 (2008-03-13) * figures 1-4 * * abstract * * page 1, line 10 - line 14 * * page 2, line 8 - line 10 * * page 2, line 17 - line 20 * * page 3, line 2 - line 10 * * page 3, line 16 - line 22 * * page 4, line 5 - line 6 * * page 5, line 5 - line 32 * * page 6, line 1 - line 6 * * claims 1-6 * ----- | 1-15 | INV. A61B5/053 |
| X A | US 2008/218180 A1 (WAFFENSCHMIDT EBERHARD [DE] ET AL) 11 September 2008 (2008-09-11) * claims 1-14 * * figure 2a * ----- | 1,11 2-10, 12-15 | |
| A | WO 2006/111877 A (PHILIPS INTELLECTUAL PROPERTY [DE]; KONINKL PHILIPS ELECTRONICS NV [NL] 26 October 2006 (2006-10-26) * the whole document * ----- | 1-15 | TECHNICAL FIELDS SEARCHED (IPC) A61B |

The present search report has been drawn up for all claims

| Place of search | Date of completion of the search | Examiner |
|---|---|---|
| The Hague | 9 September 2009 | De la Hera, Germán |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
............................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 09 16 5509

This annex lists the patent family members relating to the patent documents cited in the above-mentioned European search report.
The members are as contained in the European Patent Office EDP file on
The European Patent Office is in no way liable for these particulars which are merely given for the purpose of information.

09-09-2009

| Patent document cited in search report | | Publication date | Patent family member(s) | | Publication date |
|---|---|---|---|---|---|
| WO 2008029316 | A | 13-03-2008 | NONE | | |
| US 2008218180 | A1 | 11-09-2008 | CN | 101218513 A | 09-07-2008 |
| | | | WO | 2007007217 A1 | 18-01-2007 |
| | | | JP | 2009501040 T | 15-01-2009 |
| WO 2006111877 | A | 26-10-2006 | CN | 101160093 A | 09-04-2008 |
| | | | JP | 2008536606 T | 11-09-2008 |

EPO FORM P0459

For more details about this annex : see Official Journal of the European Patent Office, No. 12/82